(19) Europäisches Patentamt
European Patent Office
Office européen des brevets

(11) **EP 3 382 647 A1**

(12) **EUROPEAN PATENT APPLICATION**

(43) Date of publication:
**03.10.2018 Bulletin 2018/40**

(51) Int Cl.:
***G06T 11/20*** *(2006.01)*

(21) Application number: **17164067.5**

(22) Date of filing: **31.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **WEKEL, Tilman**
**5656 AE Eindhoven (NL)**
• **WEESE, Juergen**
**5656 AE Eindhoven (NL)**
• **CAROLUS, Heike**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **DEVICE AND METHOD FOR SKETCH TEMPLATE GENERATION OR ADAPTION**

(57) The present invention relates to a device and method for sketch template generation or adaption. To provide an efficient link between the image data and the template sketch, the device comprises a model input (40) configured to obtain a patient-adapted anatomical model, a parameter extraction unit (41) configured to extract a set of model parameters from the patient-adapted model, and a sketch unit (42) configured to generate or adapt a sketch template based on the extracted set of model parameters.

FIG. 2

## Description

FIELD OF THE INVENTION

The present invention relates to a device and method for sketch template generation or adaption. The present invention relates further to a medical system comprising such a device.

BACKGROUND OF THE INVENTION

Sketch templates are important tools for visual reporting. A report for diagnosis or a follow up usually consists of text and graphical items. Sketch templates allow the clinician to quickly visualize conditions or observations in simplified drawings, either by hand or automatically based on software applications. A typical example is a chamber view that is used to report wall motion artifacts. The sketch is derived from a slice view of the heart chamber. Sketches generally show some level of abstraction with no direct relation to the anatomy of a specific patient.

A sketch may generally be understood as a simplified drawing of an anatomical part/region that emphasizes specific features or aspects in a comprehensive manner. A sketch template may generally be understood as a predefined sketch that is used for patient specific annotations marked or edited by the clinician. A sketch template is often simply called "sketch" as well.

Sketch templates are mainly used and populated manually. In many cases, the clinician manually marks findings in the sketch on a piece of paper. These findings are derived from images or other patient-specific data (previous reports etc.). Recently, there are applications available that automatize this process by providing interactive and parameterized tools to create and populate sketches electronically. However, there are two essential problems in this workflow.

The first problem is that there is a gap between medical imagery and visual reporting. Despite the various tools and algorithms for the processing of medical images, there is no direct link to visual reporting. The clinician might be assisted in interpreting and classifying medical images, but transferring this information onto the sketch templates or visual reports is often done manually.

The second problem is that common sketches are not personalized to patient data and that they are not adaptable to the patient data simply because they are represented by fixed drawings. This makes it quite complicated to identify the correct anatomical body sites and link them to what is seen in the image data. The inter-patient variability of anatomical structures can be quite high.

US 2011/0169864 A1 discloses a system for producing a representation of an object in image data, based on a template coupled to a model of the object, the system comprising a model unit for adapting the model to the object in the image data, and a template unit for adapting the template to the adapted model on the basis of the coupling between the template and the model. The template defines a representation of the object which is simpler to interpret than the model. The template may be arranged to emphasize useful features of the object. The template comprises substantially fewer degrees of freedom and thus can be efficiently adapted to the model. Because the template is coupled to the model, the position, orientation and/or shape of the template is determined by the model adapted to the object in the image data. Hence, the template is adapted to the image data. The adapted template is capable of representing the object and its individual characteristics, e.g., the shape of the object as well as the position and/or orientation of the object with respect to an external reference system defined, e.g., based on the image data.

The solution disclosed in this document does, however, also not resolve the above mentioned problems and does particularly not show a way to provide an efficient link between the image data and the template sketch.

SUMMARY OF THE INVENTION

It is an object of the present invention to provide a device and method for sketch template generation or adaption as well as a corresponding medical system, which resolve the above-mentioned problems and particularly provide an efficient link between the image data and the template sketch.

In a first aspect of the present invention a device for sketch template generation or adaption is presented comprising

- a model input configured to obtain a patient-adapted anatomical model,
- a parameter extraction unit configured to extract a set of model parameters from the patient-adapted model, and
- a sketch unit configured to generate or adapt a sketch template based on the extracted set of model parameters.

In a further aspect of the present invention a medical is presented comprising

- an imaging apparatus for acquiring medical image data of a patient's region of interest,
- a model adaption unit configured to adapt an anatomical model of anatomy in said region of interest based on the obtained medical image data, and
- a device as disclosed herein for sketch template generation or adaption based on the adapted anatomical model.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to automatically compute patient-specific sketch templates, e.g. to newly generate a sketch template or adapt a give sketch template based on patient imagery data. For instance, in the field of chamber view sketch templates the invention may advantageously be applied. Depending on the complexity of the model, the sketch template is personalized at different levels of abstraction. Hence, according to the present invention a sketch template is not static but can be adapted, e.g. by a set of parameters resulting in a parametrized sketch template. If the data, i.e. the model parameters that can be extracted from the patient-adapted model, is noisy or contains only little information, the sketch template can be customized with a very limited set of parameters. If the data allows fitting more complex models, the sketch template can also be generated procedurally which increases the level of adaption and makes the adaption independent of a predefined set of possible sketch configurations.

The connection between adapted models and sketch templates yields a link between (image) data domain and sketch templates. Clinical findings or observations that are derived from the image data can thus easily be mapped to the sketch template.

Further, the user, e.g. a physician such as a radiologist, can select one among a given set of sketch templates which is then automatically adapted to the data. The user can easily change rendering or style properties such as color, texture or line thickness.

In an embodiment said parameter extraction unit is configured to extract a set of model parameters from the patient-adapted model that correspond to or are related to sketch template parameters of the sketch template to be generated or adapted. This allows an improved adaption or generation of a sketch template if the appropriate model parameters are extracted from the model rather than model parameters that cannot be used for adaptation or generation of a sketch template.

In another embodiment said parameter extraction unit is configured to analyze the sketch template to be generated or adapted to extract the sketch template parameters and to determine the set of model parameters from the extracted sketch template parameters. This also improves the extraction of model parameters from the model that are useful in adapting / generating a sketch template.

In another embodiment said parameter extraction unit is configured to extract sketch template information from the model, the sketch template information indicating one or more sketch templates that can be generated or adapted based on model parameters of said model and wherein said sketch unit is configured to generate or adapt a sketch template from the one or more sketch templates indicated by the extracted sketch template information.

In a similar embodiment the parameter extraction unit is configured to extract sketch template information from the model, the sketch template information indicating one or more sketch template parameters which correspond or are related to model parameters of said model, and to extract one or more model parameters from the patient-adapted model that correspond to or are related to the one or more sketch template parameters indicated by the extracted sketch template information.

Thus, in embodiments the present invention proposes to encode sketch templates into the models in order to establish a link between data-driven analysis and visual reporting. Depending on the complexity of the model, the sketch as well as its parameters can be encoded at different levels of abstraction / simplicity. For instance, the encoding allows generating or deriving the sketch from the adapted model in a straight forward manner.

The sketch templates may e.g. be encoded as a set of parameters that allow a procedural rendering given the model. For instance, within cardiac reporting, many sketches are based on a slice view of the heart model. The corresponding slice plane can be encoded based on the integrated landmarks and labels. A stylesheet may then be applied to apply colors, fonts, or other rendering properties.

A stylesheet is a set of parameters that describe how the sketch is visualized. It typically instantiates a predefined format. The format has a hierarchical structure. The parameters are associated to geometric sketch primitives such as lines, curves or text elements. All elements can have a shadow, i.e. a line can have a thickness, a solid object can have a filling color, etc.

The parameter extraction unit may further be configured to extract model parameters including one or more of segment identifiers, relative or absolute length information, relative or absolute area information, position information, and aspect ratio. This further improves the generation and adaption of sketch templates.

The sketch unit may be configured in an embodiment to generate or adapt a sketch template using one or more transformation rules, in particular comprising geometric operations, said transformation rules indicating the relationship between one or more model parameters and one or more sketch template parameters of the sketch template to be generated or adapted. Said transformation rules may be predetermined based on the relationship between known models and sketch templates. Hence, once the model and the sketch template are known, the corresponding transformation rules maybe selected and used to generated or adapt the sketch template.

For instance, geometric operations such as multiplications of size parameters (length, width, area, angle, etc.) by a factor may be performed as such a transformation. A geometric operation can also be non-linear or even discrete transformation. For instance, there are different types of morphological abnormalities that require completely different sketches. This could not be reflected by a simple (linear) transformation because it is not just a question of different sizes or scaling but of a completely different topology. The adapted heart model could be classified to belong to one of a finite set of different anomalies and the sketch template could be selected accordingly. The classification could be done based on a set of measurements that are extracted from the adapted heart model. Each class is then associated with a different sketch template.

In an embodiment said sketch unit is configured to generate or adapt a sketch template using one or more landmarks available in the patient-adapted model and the sketch template. Such landmarks, e.g. anatomical features, maybe selected by a user and/or may be automatically determined, e.g. based on image processing tools as commonly known in the art. By use of such landmarks the relationship between the patient-adapted model and the sketch template can be determined, which helps in the generation or adaption of a sketch template.

The device may further comprise a user input configured to obtain user input, said user input indicating a sketch template to be generated or adapted, wherein said parameter extraction unit is configured to extract a set of model parameters from the patient-adapted model that correspond to or are related to sketch template parameters of the sketch template indicated by the user input, and/or said user input indicating modifications of the sketch template. Hence, the user can actively influence the generation / adaption of a sketch template.

In another embodiment the device further comprising a model adaption unit configured to adapt an anatomical model of anatomy in a patient's region of interest based on obtained medical image data of the patient's region of interest. The adaption of a model based on patient data is generally known and e.g. described in Ecabert, Olivier, et al. "Automatic model-based segmentation of the heart in CT images." IEEE transactions on medical imaging 27.9 (2008): 1189-1201.

The sketch unit may be configured to transfer meta-information, in particular labels, annotations, findings, transfer observations and/or measurements, from the patient-adapted model to the generated or adapted sketch template. This is enabled by the present invention according to which the link between the patient data / the patient-adapted model and the sketch template is finally known. This link can thus be exploited to transfer such meta-information from the patient-adapted model to the generated or adapted sketch template.

In a further embodiment said sketch unit is configured to generate or adapt a sketch template based on the accuracy, resolution and/or amount of model parameters that can be extracted from the patient-adapted model and/or based on the accuracy of the adaptation of the model to medical image data of the patient. Hence, if e.g. more (in numbers) and/or more accurate model parameters are known the adaption/generation of the sketch template can be made more detailed.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of an embodiment of a medical system according to the present invention,
Fig. 2 shows a schematic diagram of an embodiment of a device according to the present invention,
Fig. 3 shows different sketch templates that may be used according to the present invention,
Fig. 4 shows a perspective view of a general model of the heart and a slice view of a corresponding patient-adapted model,
Fig. 5 shows a diagram illustrating the link between patient-adapted model and sketch template as used according to the present invention,
Fig. 6 shows a diagram of a mesh model indicating measurements of model parameters,
Fig. 7 illustrates ontology-based linking between model parameters and sketch template parameters,
Fig. 8 illustrates the encoding of a sketch template into a digital anatomical model, and
Fig. 9 illustrates a semantic abstraction layer linking a model and a sketch template.

DETAILED DESCRIPTION OF THE INVENTION

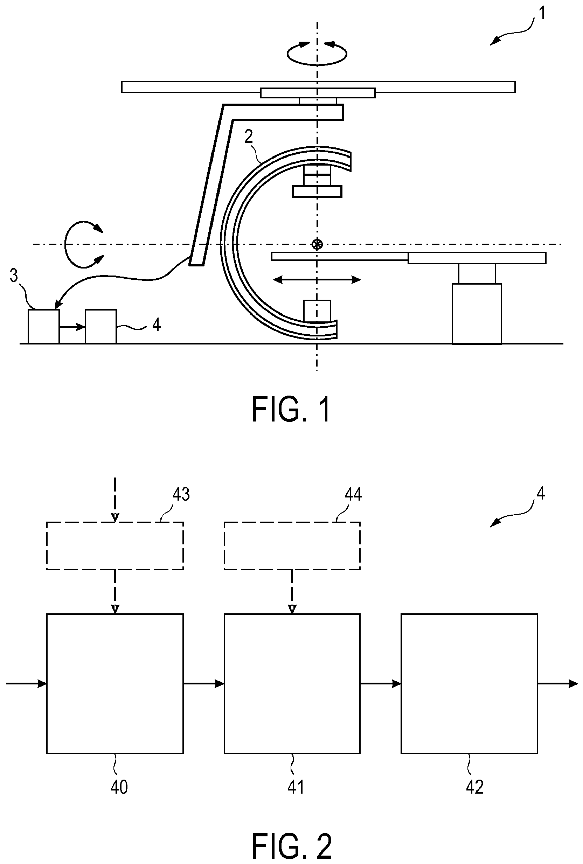

Fig. 1 shows a schematic diagram of an embodiment of a medical system 1 according to the present invention. In this embodiment the medical system 1 comprises an imaging apparatus 2 for acquiring medical image data of a patient's region of interest, e.g. of the patient's heart. The imaging apparatus 2 is, in this example, an X-ray imaging apparatus. In other non-limiting embodiments, the imaging apparatus may be a fluoroscopic imaging device, for example, a computed tomography X-ray device, a fluoroscopic low-dose X-ray device, or an angiographic imaging device.

The medical system 1 further comprises a model adaption unit 3 configured to adapt an anatomical model of anatomy in said region of interest based on the obtained medical image data. Further, the medical system 1 comprises a device for sketch template generation or adaption based on the adapted anatomical model. The model adaption unit 3 may also be part of the device 4. The model adaption unit 3 and/or the device 4 may be implemented in soft- and/or hardware, e.g. as a computer program running on a PC, processor or workstation.

Fig. 2 shows a schematic diagram of an embodiment of a device 4 for sketch template generation or adaption according to the present invention. In this embodiment the device 4 comprises a model input 40 configured to obtain a patient-adapted anatomical model, a parameter extraction unit 41 configured to extract a set of model parameters from the patient-adapted model, and a sketch unit 42 configured to generate or adapt a sketch template based on the extracted set of model parameters.

Optionally, further elements are provided, such as a model adaption unit 43 configured to adapt an anatomical model of anatomy in a patient's region of interest based on obtained medical image data of the patient's region of interest. Said model adaption unit 43 may functionally be identical to the model adaption unit 3 provided as separate element in the medical system 1 shown in Fig. 1.

Further, a user input 44 configured to obtain user input may be provided as another optional unit. Said user input may indicate a sketch template to be generated or adapted. In this case the parameter extraction unit 41 may be configured to extract a set of model parameters from the patient-adapted model that correspond to or are related to sketch template parameters of the sketch template indicated by the user input, and/or said user input indicating modifications of the sketch template.

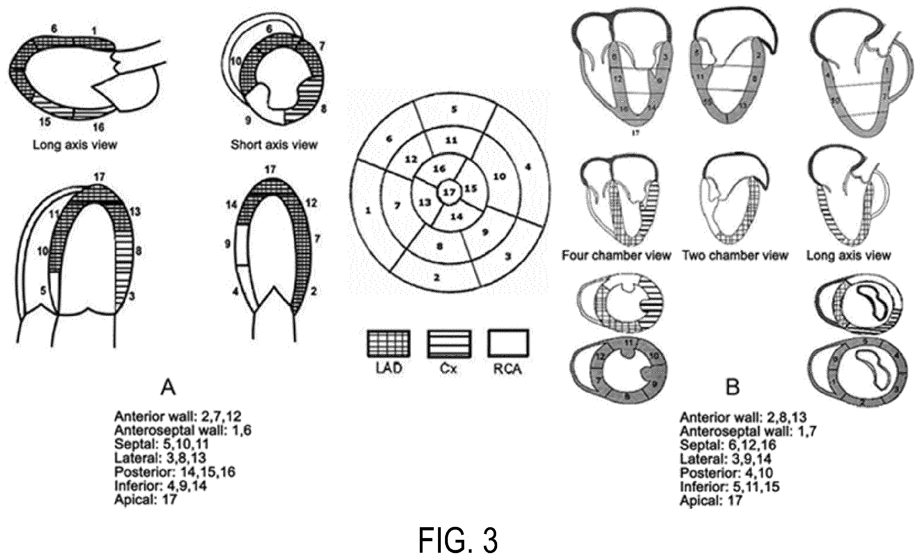

Fig. 3 shows different sketch templates including an AHA segment model (also called 17 segment model, shown in the center of Fig. 3) that may be used according to the present invention. Sketch templates are an important tool for visual reporting. A report for diagnosis or follow ups usually consists of text and graphical items. Sketch templates allow the clinician to quickly visualize conditions or observations in simplified drawings, either by hand or automatically based on software applications. A typical example is the chamber view that is used to report wall motion artifacts as shown in Fig. 3. The sketch template is derived from a slice view of the heart chamber. As it can be seen in both examples, the sketches show some level of abstraction with no direct relation to the anatomy of a specific patient.

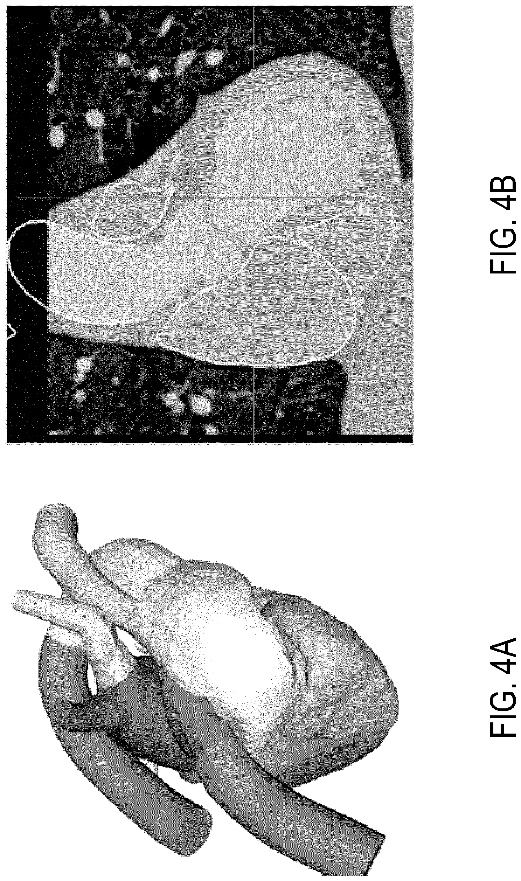

Hand-crafted, mesh-based models typically comprise labeled vertices and triangles which can be associated with landmarks, semantic labels and measurements. Since the topology does not change during the adaption step, this type of information can be mapped from an initial model shown in Fig. 4A to the patient-specific domain, shown in Fig. 4B as slice view of a corresponding patient-adapted model, and vice versa.

Anatomical models are typically given as a set of geometric entities, such as meshes, points, geometric primitives or higher order surface models. These entities are assigned with meta-information such as physical properties and anatomical labels. In the following, two approaches are described to encode sketch templates into digital anatomical models. The adaption based on parameter encoding assumes the sketch to have a limited number of parameters that can be extracted from the (adapted) model. This approach is quite scalable in terms of model complexity. If the model is very simple, the number of model parameters is reduced to a minimum. Procedural encoding means that the sketch template is not given by a parametrized template but as a "procedure" that describes how to generate the sketch template from the model by applying geometric operations.

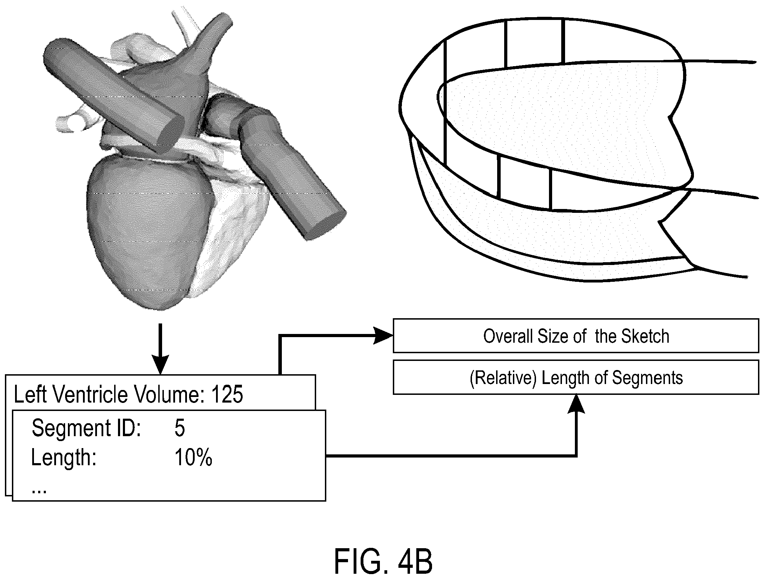

An example of such a geometric operation is using the aspect ratio of the sketch template as illustrated in Fig. 5. The ratio of height and width of the sketch template (SV) corresponds linearly with the ratio of volume (VLV) to area of the largest cross-section ALV) of the left ventricle in the 3D model:

$$SV = ALV / VLV.$$

SV would then be multiplied with the absolute width (given e.g. in pixels) in order to obtain the height of the sketch template.

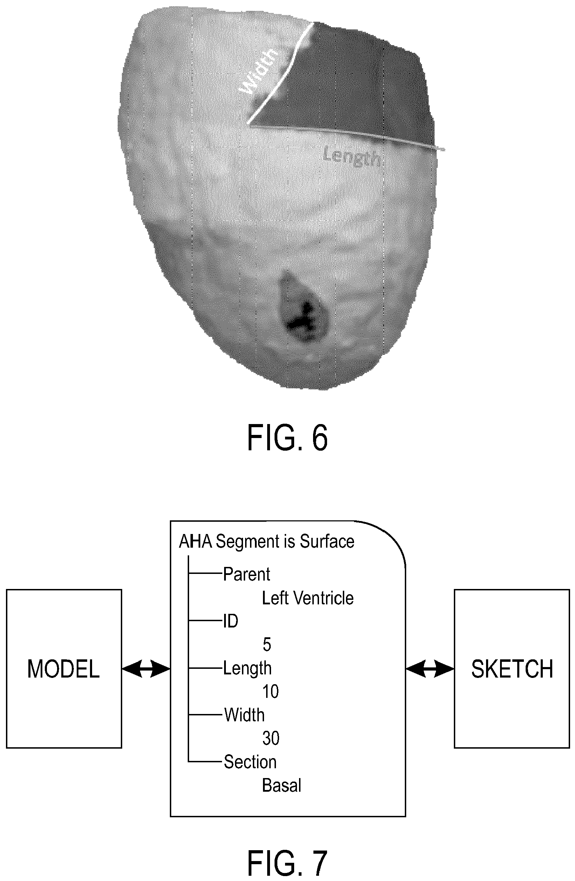

An example of such a geometric operation is using the relative length of the AHA segments as illustrated in Fig. 6. The segments are coded on the surface of the 3D heart model. The model is represented by a triangulated mesh. Each triangle hence carries a label to which AHA segment it belongs. In this way the area of each AHA segment (AS) can be easily calculated after adaption to the patient data. Further, also the area of the complete surface of the left ventricle (AOLV) can be easily calculated. The relative length of the segments (RLS) in the sketch template could now result as follows:

$$RLS = AS / AOLV.$$

The relative length can now be multiplied by an absolute (pixel based) length in order to obtain a concrete number.

In the following, an embodiment for parameter encoding shall be described. The sketch template is given by a geometric/text-based representation that has a set of sketch template parameters allowing the configuration and user-adaptation of the sketch template. As shown in Fig. 5, a view of the left heart chamber is given as a predefined sketch template. It has a different set of sketch template parameters for different degrees of abstraction. In a very simple version, the sketch template can only be customized by its size. If there is more data (i.e. model parameters) available, the size of the wall segments can be parametrized. The corresponding parameters are encoded into a heart model (shown on the left hand side in Fig. 5) such that they can directly be inferred once the model has been fitted to the data.

The AHA segments may be encoded onto the mesh of the model, as shown in Fig. 6, in order to measure e.g. their width and height. This data can be used to parametrize the corresponding segment lengths in the chamber view sketch template as shown on the right hand side in Fig. 5). Fig. 5 thus illustrates the link between patient-adapted model and sketch template as used according to the present invention and Fig. 6 shows a diagram of a mesh model indicating measurements of model parameters.

In an embodiment the sketch template parameters are encoded based on a structured, semantic format shown in Fig. 7. The central box is an instance of a predefined class AHA segment. Classes are defined within an ontology such as SNOMED CT, currently described e.g. at https://www.nlm.nih.gov/healthit/snomedct/index.html.

In a medical context, an ontology is basically a set of clinical terms represented in a hierarchical, semantic structure. Among many others, the ontology covers anatomy, findings, and procedures. According to the present invention, the subset that describes the anatomy is used to establish a semantic link between model and sketch. The ontology describes a body-site on a very abstract level, such as "an AHA segment 'is a' surface and 'is located' on the left ventricular wall", "the left ventricular wall 'is part' of the heart, etc. This abstract representation also allows defining "locations" in a geometrical sense. Given the type of geometric primitive, surface, the location is uniquely defined by two coordinates u and v. In the adapted model domain, the "surface" is given as a set of triangles in 3D space, while it might be a closed 2D contour in the sketch. At the level of the abstract description, driven by ontology, they are the same.

The structured format thus allows defining a unified interface between model and sketch elements. The class "AHA segment" inherits ("is-a" relationship) from the geometrical model surface. This clearly defines how a "location" on this entity is defined. For a surface patch, it is given by u and v coordinates.

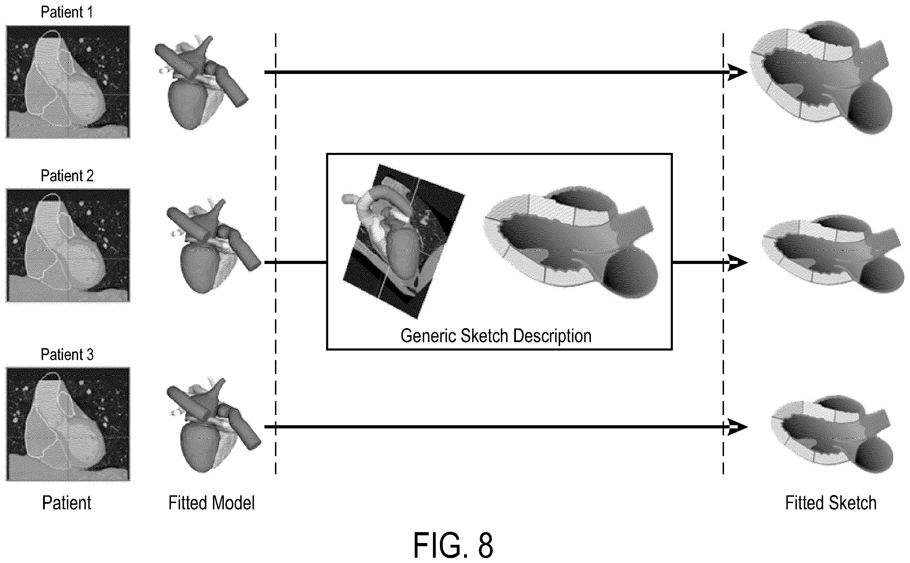

In another embodiment procedural encoding may be used. Instead of just configuring a pre-defined sketch template, the sketch template can also be inferred directly from the model based on a procedural description as illustrated in Fig. 8. The long axis view for the annotation of wall motion artefacts could be generated by the following procedure:

1. Define a suitable slice plane for a long axis view based on landmarks encoded into the model.

2. Compute the contour lines resulting from intersecting the plane with the 3D heart model.

3. The label of each contour is inferred from the label of the corresponding 3D structure in the 3D model.

4. Apply style properties such as line width, transparency, gradients or fill patterns to the resulting set of contours and solids.

In order to construct the sketch template from the model, the following information needs to be encoded into the model:

i) A set of landmarks that allows to uniquely infer a slice plane. For example, the center point of the ventricular septum, the apex (point encoded onto the mesh) and the surface normal of the ventricular septum would be sufficient to define the plane. The surface normal of the ventricular septum should lie in the plane.

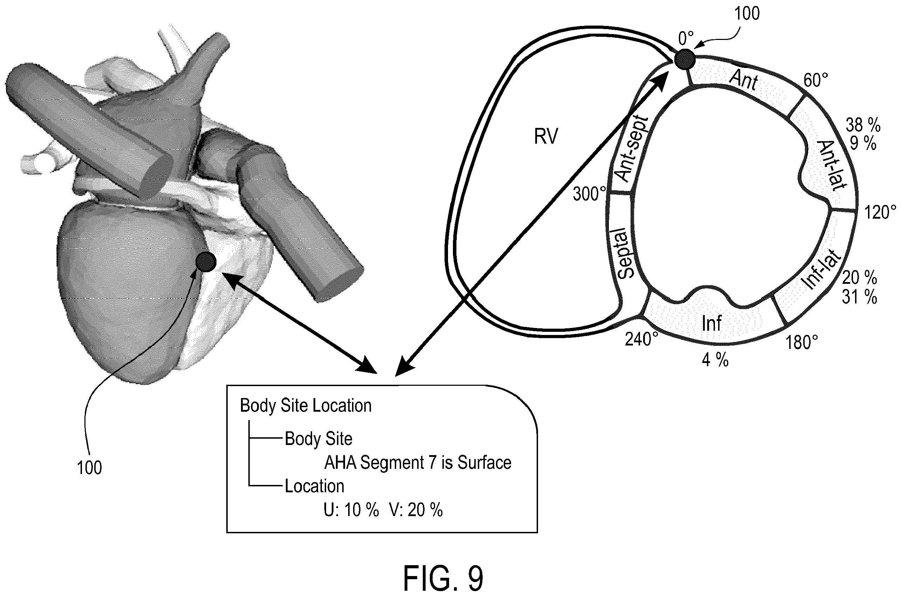

ii) The anatomical parts that will be visible in the sketch are associated with a body-site node in the ontology, e.g. an ontology as illustrated in Fig. 9. This is needed to associate semantic information with the resulting geometric entities in the sketch template. The link also allows the encoding and mapping of locations since the anatomical elements inherit geometric classes.

The advantage of this approach is that it allows to fully customize/personalize the sketch template in terms of shape and perspective. However, it requires the model to contain all geometrical details that will be part of the sketch template.

An additional feature of the proposed encoding of sketch templates is to establish a spatial registration between the data domain (e.g. a CT volume or an ultrasound image or an MRT image) and the sketch template. For both approaches described above (i.e. the procedural and parameter encoding), sketch template and anatomical model meet at an abstract/semantic representation of a body site, as shown in Fig. 9. This is implemented based on structured semantic labels (ontology) and geometric primitives. For instance, an AHA segment in the model (as shown in Fig. 9 on the left hand side) as well as an AHA segment in the sketch template (as shown in Fig. 9 on the right hand side) inherits a surface. The position on a surface can uniquely be described by two parameters, u and v. This interface (i.e. the dot 100 in Fig. 9) allows mapping a location from the model to the sketch domain.

According to embodiments of the present invention a hierarchic adaption of a sketch template is enabled, i.e. that a sketch template can parametrized and adapted on different levels of abstraction or levels of detail. Depending e.g. on the resolution of the patient data and the related accuracy of the model less or more model parameters can be extracted. If no model parameters exist or if they can be measured with a too low accuracy, the statistic mean value of this parameter can be used as default solution.

For example, in a simple case, as illustrated in Fig. 5, only the ratio between width and length of the sketch template and be adapted, e.g. the thick ventricle vs. the narrow long ventricle. Relative lengths of the single AHA segments would then all be equal or equal to the respective statistic mean value, respectively. If the model were adaptable with sufficient accuracy (given by a high resolution of the triangles of the mesh model and low failure variances), also the areas of the AHA segment could be used in order to adapt them on the relative lengths in the 2D view of the sketch template.

In another example as illustrated in Fig. 8, in a simple case the contour of the left and right ventricle is simple or corresponds to the statistically determined form. Only the size ratio of left and right ventricle (LV, RV) would then be adapted. A model with higher resolution would allow depicting also the exact path of the ventricle's wall in the sketch template.

Further, according to embodiments of the present invention it is both possible to link arbitrary sketch templates via the intermediate step of the ontology (abstraction layer) and to configure the sketch template completely freely using the procedural encoding described above (e.g. to vary the angle of intersection for the 4-chamber view, to modify the line thickness, colors, etc.). This is particularly relevant in order to "translate" the annotations correctly from the patient data and/or the patient-adapted model into the sketch template.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

**1.** Device for sketch template generation or adaption, said device comprising:

- a model input (40) configured to obtain a patient-adapted anatomical model,
- a parameter extraction unit (41) configured to extract a set of model parameters from the patient-adapted model, and
- a sketch unit (42) configured to generate or adapt a sketch template based on the extracted set of model parameters.

**2.** Device as claimed in claim 1, wherein said parameter extraction unit (41) is configured to extract a set of model parameters from the patient-adapted model that correspond to or are related to sketch template parameters of the sketch template to be generated or adapted.

**3.** Device as claimed in claim 2, wherein said parameter extraction unit (41) is configured to analyze the sketch template to be generated or adapted to extract the sketch template parameters and to determine the set of model parameters from the extracted sketch template parameters.

**4.** Device as claimed in claim 1, wherein said parameter extraction unit (41) is configured to extract sketch template information from the model, the sketch template information indicating one or more sketch templates that can be generated or adapted based on model parameters of said model and wherein said sketch unit (42) is configured to generate or adapt a sketch template from the one or more sketch templates indicated by the extracted sketch template information.

**5.** Device as claimed in claim 1, wherein said parameter extraction unit (41) is configured to extract sketch template information from the model, the sketch template information indicating one or more sketch template parameters which correspond or are related to model parameters of said model, and to extract one or more model parameters from the patient-adapted model that correspond to or are related to the one or more sketch template parameters indicated by the extracted sketch template information.

**6.** Device as claimed in claim 1, wherein said parameter extraction unit (41) is configured to extract model parameters including one or more of segment identifiers, relative or absolute length information, relative or absolute area information, position information, and aspect ratio.

**7.** Device as claimed in claim 1, wherein said sketch unit (42) is configured to generate or adapt a sketch template using one or more transformation rules, in particular comprising geometric operations, said transformation rules indicating the relationship between one or more model parameters and one or more sketch template parameters of the sketch template to be generated or adapted.

8. Device as claimed in claim 1, wherein said sketch unit (42) is configured to generate or adapt a sketch template using one or more landmarks available in the patient-adapted model and the sketch template.

9. Device as claimed in claim 1, further comprising a user input (44) configured to obtain user input, said user input indicating a sketch template to be generated or adapted, wherein said parameter extraction unit (41) is configured to extract a set of model parameters from the patient-adapted model that correspond to or are related to sketch template parameters of the sketch template indicated by the user input, and/or said user input indicating modifications of the sketch template.

10. Device as claimed in claim 1, further comprising a model adaption unit (43) configured to adapt an anatomical model of anatomy in a patient's region of interest based on obtained medical image data of the patient's region of interest.

11. Device as claimed in claim 1, wherein said sketch unit (42) is configured to transfer meta-information, in particular labels, annotations, findings, transfer observations and/or measurements, from the patient-adapted model to the generated or adapted sketch template.

12. Device as claimed in claim 1, wherein said sketch unit (42) is configured to generate or adapt a sketch template based on the accuracy, resolution and/or amount of model parameters that can be extracted from the patient-adapted model and/or based on the accuracy of the adaptation of the model to medical image data of the patient.

13. Medical system comprising:

   - an imaging apparatus (2) for acquiring medical image data of a patient's region of interest,
   - a model adaption unit (3) configured to adapt an anatomical model of anatomy in said region of interest based on the obtained medical image data, and
   - a device (4) as claimed in claim 1 for sketch template generation or adaption based on the adapted anatomical model.

14. Method for sketch template generation or adaption, said method comprising:

- obtaining a patient-adapted anatomical model,
- extracting a set of model parameters from the patient-adapted model, and
- generating or adapting a sketch template based on the extracted set of model parameters.

15. Computer program comprising program code means for causing a computer to carry out the steps of the method as claimed in claim 14 when said computer program is carried out on the computer.

FIG. 1

FIG. 2

Long axis view

Short axis view

Four chamber view

Two chamber view

Long axis view

LAD    Cx    RCA

A

Anterior wall: 2,7,12
Anteroseptal wall: 1,6
Septal: 5,10,11
Lateral: 3,8,13
Posterior: 14,15,16
Inferior: 4,9,14
Apical: 17

B

Anterior wall: 2,8,13
Anteroseptal wall: 1,7
Septal: 6,12,16
Lateral: 3,9,14
Posterior: 4,10
Inferior: 5,11,15
Apical: 17

FIG. 3

FIG. 4B

FIG. 4A

Left Ventricle Volume: 125

Segment ID:     5
Length:         10%
...

Overall Size of the Sketch

(Relative) Length of Segments

FIG. 4B

EP 3 382 647 A1

FIG. 6

FIG. 7

Patient 1

Patient 2

Patient 3

Patient

Fitted Model

Generic Sketch Description

Fitted Sketch

FIG. 8

RV

100

0°

Ant 60°

Ant-sept

300°

Septal

240°

Inf

4 %

180°

Inf-lat

20 %
31 %

120°

Ant-lat

38 %
9 %

100

Body Site Location

├── Body Site
│       AHA Segment 7 is Surface
└── Location
        U: 10 %  V: 20 %

FIG. 9

EP 3 382 647 A1

Europäisches
Patentamt

European
Patent Office

Office européen
des brevets

# EUROPEAN SEARCH REPORT

Application Number

EP 17 16 4067

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2011/169864 A1 (LORENZ CRISTIAN [DE] ET AL) 14 July 2011 (2011-07-14) * paragraphs [0011], [0034] - [0040], [0047], [0051] - [0053]; figure 1 * ----- | 1-15 | INV. G06T11/20 |
| A | WEI CHEN ET AL: "Shape Context Preserving Deformation of 2D Anatomical Illustrations", COMPUTER GRAPHICS FORUM, vol. 28, March 2009 (2009-03), pages 1-12, XP055078857, DOI: 10.1111/j.1467-8659.2008.01300.x * abstract; figures 2,4,9 * * sections 4.1 and 4.2 * ----- | 1-15 | |
| A | WO 2015/166377 A1 (KONINKL PHILIPS NV [NL]) 5 November 2015 (2015-11-05) * abstract; figures 2,3 * * paragraphs [0032] - [0033] * ----- | 1-15 | |
| A | US 2016/267626 A1 (ALLAIRE STÉPHANE [FR] ET AL) 15 September 2016 (2016-09-15) * abstract; figures 4-6 * ----- | 1-15 | TECHNICAL FIELDS SEARCHED (IPC) G06T G06F |

The present search report has been drawn up for all claims

| Place of search | Date of completion of the search | Examiner |
|---|---|---|
| Munich | 30 May 2017 | Krawczyk, Grzegorz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**EP 3 382 647 A1**

## ANNEX TO THE EUROPEAN SEARCH REPORT
## ON EUROPEAN PATENT APPLICATION NO.

EP 17 16 4067

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2017

| Patent document cited in search report | | Publication date | Patent family member(s) | | Publication date |
|---|---|---|---|---|---|
| US 2011169864 | A1 | 14-07-2011 | CN | 102272797 A | 07-12-2011 |
| | | | EP | 2332124 A2 | 15-06-2011 |
| | | | JP | 5602142 B2 | 08-10-2014 |
| | | | JP | 2012507066 A | 22-03-2012 |
| | | | RU | 2011116411 A | 10-11-2012 |
| | | | US | 2011169864 A1 | 14-07-2011 |
| | | | WO | 2010035183 A2 | 01-04-2010 |
| WO 2015166377 | A1 | 05-11-2015 | CN | 106456125 A | 22-02-2017 |
| | | | EP | 3136972 A1 | 08-03-2017 |
| | | | US | 2017124771 A1 | 04-05-2017 |
| | | | WO | 2015166377 A1 | 05-11-2015 |
| US 2016267626 | A1 | 15-09-2016 | NONE | | |

EPO FORM P0459

For more details about this annex : see Official Journal of the European Patent Office, No. 12/82

**EP 3 382 647 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110169864 A1

**Non-patent literature cited in the description**

- **ECABERT, OLIVIER et al.** Automatic model-based segmentation of the heart in CT images. *IEEE transactions on medical imaging,* 2008, vol. 27 (9), 1189-1201